# EUROPEAN PATENT APPLICATION

(11) **EP 4 802 984 A1**
(43) Date of publication of application: **09.09.2026**
(21) Application number: 26162032.2
(22) Date of filing: 03.03.2026
(51) Int. Cl.: A61B 1/00, A61B 1/12

(54) **NOVEL ENDOSCOPIC CLEANING ADAPTER**

(30) Priority: 07.03.2025 US 202563768311 P
(71) Applicant: GA HEALTH COMPANY LIMITED, Shatin, N.T. (HK)
(72) Inventor: MCCABE, Ken, Hong Kong (CN); CHAN, Tsan Ho, Hong Kong (CN)
(74) Representative: Ullrich & Naumann PartG mbB

(57) **Abstract**

The present invention is related to an endoscopic cleaning adapter, adaptable to position in a port of an endoscope for assisting a cleaning procedure for said endoscope, comprising: a main body, comprising an internal cavity and a limiting structure, and being positioned on said port when said valve being positioned in said port; a shaft, partially received in said internal cavity of said main body and profiled into a tubular structure having at least five recesses on the length side thereof, wherein each of said at least five recesses being fitted with a sealing member; at least two openings on the length side thereof and a hollow cavity fluidically connecting with said at least two openings defining an internal channel inside said tubular structure allowing fluid passing therethrough; and a limiting structure; a shell, adjacent said main body, for fastening said adapter to said port; characterized in that, said adapter further comprising: a cap removably engaging with said shaft and having an elongate member biasing a sealing member of a first recess of said at least five recesses of said shaft; a resilient member between said cap and said main body, whereby said shaft being adapted to be movable reciprocally between a first position where said limiting structure of said shaft engaging said limiting structure of said main body and a second position where said limiting structure of said shaft disengaging said limiting structure of said main body.

## Description

### FIELD OF THE INVENTION

The present invention is related to an endoscopic cleaning adapter, a specialized device designed for the critical reprocessing of endoscopes.

### BACKGROUND OF THE INVENTION

The current endoscopic cleaning adapter's complex design is its primary weakness. Its intricate internal channels are prone to clogging and are extremely difficult to clean perfectly, creating a risk of bacterial contamination and infection between patients. This complexity also makes the device less reliable and more expensive.

The public needs a simpler design because it directly translates to enhanced patient safety. A design with fewer, more accessible pathways is easier to sterilize thoroughly, drastically reducing infection risk. This improved reliability ensures the device functions correctly during critical procedures and lowers costs for healthcare providers, making care both safer and more affordable. Simplification, in this case, is not a compromise but a significant advancement in medical safety and efficiency.

The present invention at least seeks to address issues of these problems, or at least to provide an alternative to the public.

### SUMMARY OF THE INVENTION

The first aspect of the present invention is related to an endoscopic cleaning adapter, adaptable to position in a port of an endoscope for assisting a cleaning procedure for said endoscope, comprising: a main body, comprising an internal cavity and a limiting structure, and being positioned on said port when said valve being positioned in said port; a shaft, partially received in said internal cavity of said main body and profiled into a tubular structure having at least five recesses on the length side thereof, wherein each of said at least five recesses being fitted with a sealing member; at least two openings on the length side thereof and a hollow cavity fluidically connecting with said at least two openings defining an internal channel inside said tubular structure allowing fluid passing therethrough; and a limiting structure;; a shell, adjacent said main body, for fastening said adapter to said port; characterized in that, said adapter further comprising: a cap removably engaging with said shaft and having an elongate member biasing a sealing member of a first recess of said at least five recesses of said shaft; a resilient member between said cap and said main body, whereby said shaft being adapted to be movable reciprocally between a first position where said limiting structure of said shaft engaging said limiting structure of said main body and a second position where said limiting structure of said shaft disengaging said limiting structure of said main body.

In some embodiments, an engagement member provided to said shaft engaging with an engagement member provided to said elongate member of said cap.

In some embodiments, said shaft being manufactured as a single piece.

In some embodiments, said shaft being manufactured in plastic.

In some embodiments, said cap, said main body, and said shell being made of plastic.

In some embodiments, when said shaft being positioned in said port: when said shaft at said first position, water flow in said cleaning procedure being impeded by said adapter; and when said shaft at said second position, air flow in said cleaning procedure being impeded by said adapter.

In some embodiments, said adapter being configured to enable: when said shaft being at said first position, air flow, flowing into said port via a first conduit of said port, leaving said port via a third conduit and/or a fourth conduit of said port through fluid passage between the outer side of the surrounding wall of said shaft and the inner side of the surrounding wall of said port; and when said shaft being at said second position, water flow, flowing into said port via a second conduit of said port, leaving said port via said third conduit through said internal channel of said shaft and said fourth conduit through fluid passage between the outer side of the surrounding wall of said shaft and the inner side of the surrounding wall of said port.

In some embodiments, when said adapter being positioned in said port, and when said shaft being at said first position, a first portion of air flow flowing into said port, via said first conduit, and leaving said port via said third conduit through fluid passage between the outer side of the surrounding wall of said shaft and the inner side of the surrounding wall of said port; and/or a second portion of air flow flowing into said port, via said first conduit, and leaving said port via said fourth conduit through fluid passage between the outer side of the surrounding wall of said shaft and the inner side of the surrounding wall of said port; and/or when said shaft being at said second position, a first portion of water flow flowing into said port, via said second conduit, and leaving said port via said third conduit through said internal channel of said shaft; and/or a second portion of water flow, flowing into said port, via said second conduit, and leaving said port via said fourth conduit through fluid passage between the outer side of the surrounding wall of said shaft and the inner side of the surrounding wall of said port.

In some embodiments, said shell comprising a limiting structure collaboratively working with a limiting structure of said port for fastening said main body to said port.

In some embodiments, when said shaft being positioned in said port, said limiting structure of said main body and said limiting structure of shaft holding said shaft at least partially received in an accommodation cavity defined by said internal cavity of said main body and an internal cavity of said port of said endoscope.

In some embodiments, said at least five recesses comprising said first recess, a second recess, a third recess, a fourth recess, and a fifth recess sequentially arranged from the top to said bottom of said shaft on the length side thereof.

In some embodiments, one of said at least two openings of said shaft being provided between said second recess and said third recess, while another one of said at least two openings of said shaft being provided between said fourth recess and said fifth recess.

In some embodiments, said tubular structure being provided with at least a first opening, a second opening, a third opening, and a fourth opening, wherein said first opening and said second opening defining a first fluidic path, said third opening and said fourth opening defining a second fluidic path, said hollow cavity fluidically connecting with said first fluid path and said second fluidic path.

In some embodiments, said tubular structure being provided with at least a first opening, a second opening, a third opening, and a fourth opening, wherein said first opening and said second opening defining a first fluidic path, said third opening and said fourth opening defining a second fluidic path, said hollow cavity fluidically connecting with said first fluid path and said second fluidic path.

In some embodiments, said first fluidic path being positioned between said second recess and said third recess, while said second fluidic path being positioned between said fourth recess and said fifth recess.

The second aspect of the present invention is related to a method to provide an endoscopic cleaning adapter of the first aspect of the present invention.

### DESCRIPTION OF THE DRAWINGS

Some embodiments of the present invention will now be explained, with reference to the accompanied drawings, in which:-
- Figure 1: is a perspective view of a first embodiment of an endoscopic
- Figure 2A: cleaning adapter; is a first sectional view of the first embodiment of the endoscopic cleaning adapter;
- Figure 2B: is a second sectional view of the first embodiment of the endoscopic cleaning adapter;
- Figure 3A: is a sectional view of the first embodiment of the endoscopic cleaning adapter mounted into a port of an endoscope, in which a cap of the endoscopic cleaning adapter is unpressed; and
- Figure 3B: is a sectional view of the first embodiment of the endoscopic cleaning adapter mounted into a port of an endoscope, in which a cap of the endoscopic cleaning adapter is pressed.

### DETAILED DESCRIPTION OF PREFERRED EMBODIMENTS OF THE INVENTION

The present invention is now presented by way of examples with reference to the figures in the following paragraphs. Objects, features, and aspects of the present disclosure are disclosed in or are apparent from the following description. It should be understood by one of ordinary skilled in the art that the following description is a description of exemplary embodiments only, and is not intended as limiting the broader aspects of the present disclosure, which broader aspects are embodied in the exemplary constructions.

It should be noted that, unless otherwise defined, the technical terms or scientific terms used in the embodiments of the present invention shall have the usual meanings understood by person with ordinary skills in the art to which the present invention belongs. "First", "second" and similar expression used in the embodiments of the present invention do not indicate any order, quantity or importance, but are only used to distinguish different components. "Front", "rear", "left", "right", "upper", and "lower" and other terms indicating orientation or similar terms are only described for the exemplary relative positional relationship shown in the drawings to facilitate the understanding. It does not limit the disclosed components in the present invention can only follow this specific relative positional relationship.

For brevity's sake, the operation of cleaning an endoscope, the connection means of the endoscope to a water source, air source, and suction source are not described and explained in this specification, as this information constitute the state of art.

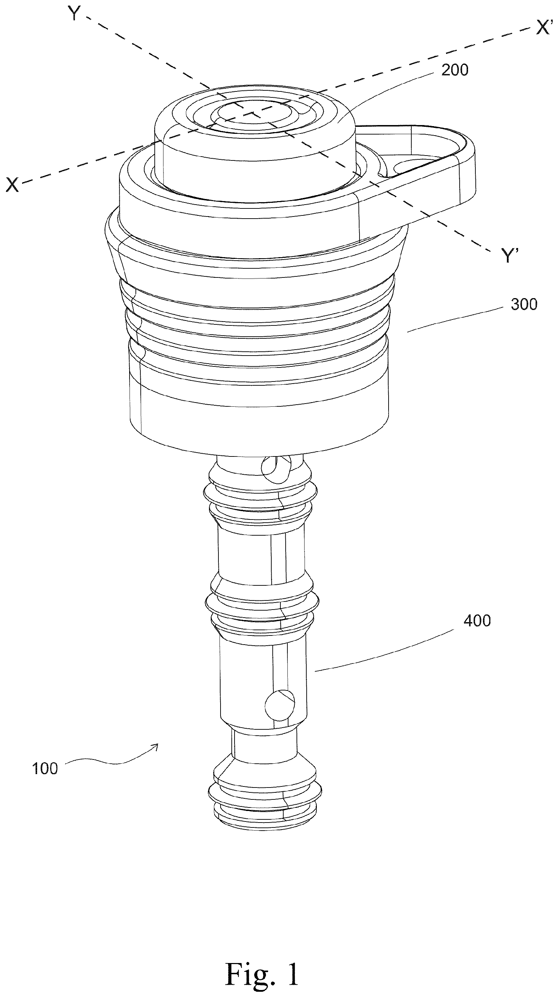

Figure 1 is a perspective view of a first embodiment of an endoscopic cleaning adapter 100, which comprises a cap 200, a shell 300, and a shaft 400.

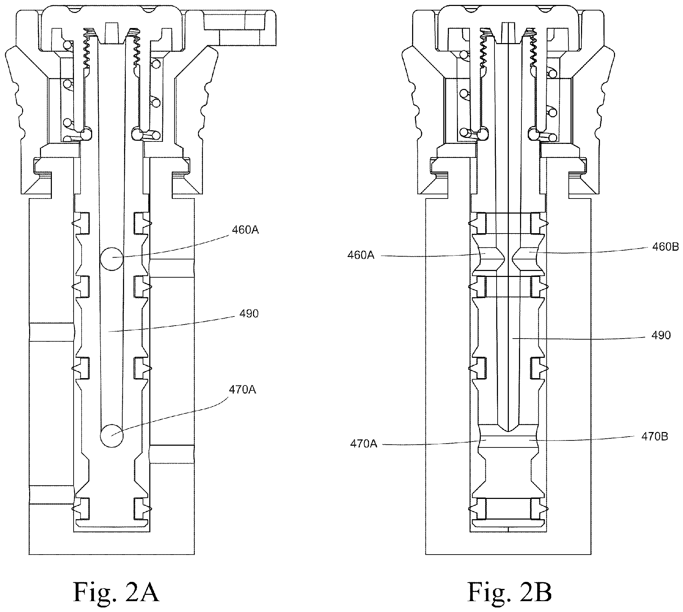

Figure 2A is a first sectional view, along dotted line XX' in figure 1, of the first embodiment of the endoscopic cleaning adapter.

Figure 2B is a second sectional view, along dotted line YY' in figure 1, of the first embodiment of the endoscopic cleaning adapter.

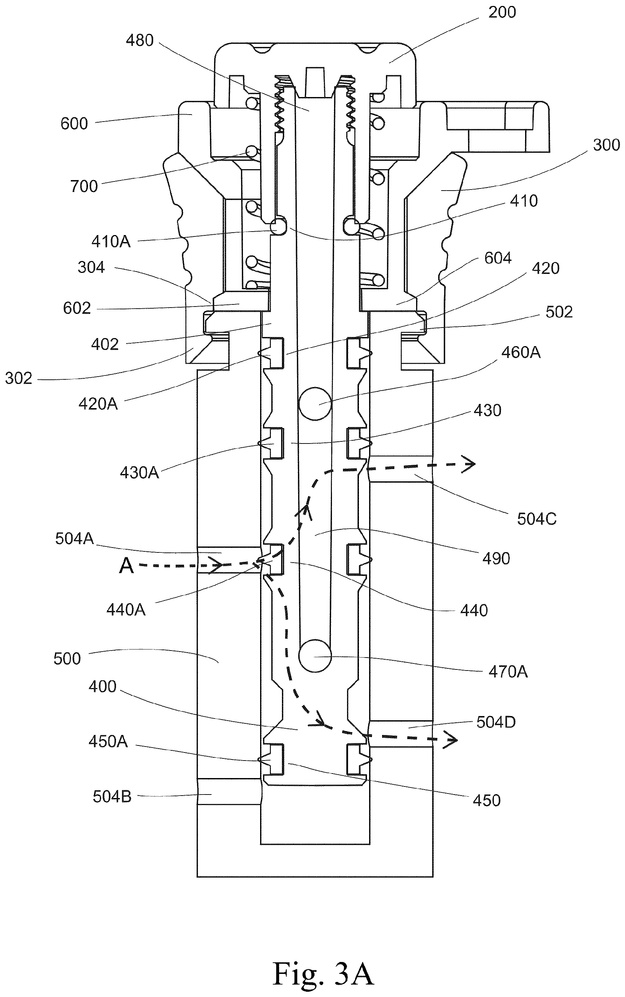

Figures 3A-3B are sectional views of the first embodiment of the endoscopic cleaning adapter 100 that is removably mounted into a port 500 which belongs to part an endoscope.

Figure 3A illustrates that the endoscopic cleaning adapter 100 further comprises a main body 600 being designed to comprise an open top and an open bottom, which defines an internal cavity within the main body 600 that allows a portion of the shaft 400 to pass through or houses a portion of the shaft 400. The main body 600 is designed to provide an inwardly protruding limiting structure 602 or 604 preferably having a right-angled shape and extending from the surrounding wall of the main body 600. It shall be important to note that the shape and/or orientation of the limiting structure 602 or 604 disclosed in figures is an example only; those skilled in the art could configure it according to actual needs.

A resilient member 700 is positioned between the cap 200 and the main body 600, in particular between the cap 200 and the main body's limiting structure 602 or 604.

With the aid of the resilient member 700, the cap 200 can move reciprocally inside the internal cavity of the main body 600 with respect to the main body 600 in a first position where the cap 200 is closer to the limiting structure 602 or 604 and a second position where the cap 200 is further from the limiting structure 602 or 604.

The cap 200 is mounted onto the upper portion the shaft 400 by means of a threaded screw, provided here as an illustrative example. For example, the cap 200 can also be mounted onto the upper portion the shaft 400 by means of snap fit and/or tight fit.

The shaft 400 has an elongate tubular structure and comprises an upper portion, a lower portion, and a limiting structure 402 outwardly protruding from the tubular structure of the shaft 400. Preferably, the limiting structure 402 is configured as a right-angled profile that is complementary to the shape of the limiting structure 602 or 604 of the main body 600. It shall be important to note that the shape and/or orientation of the limiting structure 402 disclosed in figures is an example only; those skilled in the art could configure it according to actual needs. As long as the interaction of the limiting structure 602 or 604 and the limiting structure 402 limits the movement of the shaft 400, it belongs to the gist of the limiting structure.

The interaction between the limiting structure 602 or 604 of the main body 600 and the limiting structure 402 of the shaft 400 defines the upper portion and the lower portion of the shaft 400, in which the portion of the shaft 400 above said interaction is the upper portion of the shaft 400 while the portion of the shaft 400 below said interaction is the lower portion of the shaft 400. Referring to figure 3A, the upper portion of the shaft 400 is housed inside the internal cavity of the main body 600.

The port 500 has a receptacle-like structure with an open-top internal cavity defined by the surrounding wall and bottom of the port 500. The port 500 is configured to receive the endoscopic cleaning adapter 100 and is provided with a limiting structure 502. Preferably, the limiting structure 502 is configured as a right-angled profile that is complementary to the shape of the limiting structure 602 or 604 of the main body 600 and/or the limiting structure 402 of the shaft 400. It shall be important to note that the shape and/or orientation of the limiting structure 502 disclosed in figures is an example only; those skilled in the art could configure it according to actual needs. As long as the interaction of the limiting structure 502 and the limiting structure 602 or 604 aids in the assembling between the main body 600 and the port 500, it belongs to the gist of the limiting structure.

The main body 600 is in direct contact with the port 500, as shown in figures. The main body 600 and port 500 are assembled by disposing the limiting structure 602 or 604 of the main body 600 on the limiting structure 502 of the port 500. However, the assembling between the main body 600 and the port 500 can be realized by any other means, for example, via a snug fit.

The shell 300 is profiled to provide a limiting structure 302 inwardly protruding from the peripheral of the surrounding wall of the shell 300. The shape of the limiting structure 302 is complementary to the shape of the limiting structure 502 of the port 500, which aids to assist the assembling of the shell 300 to the port 500. It shall be important to note that the shape and/or orientation of the limiting structure 302 disclosed in figures is an example only; those skilled in the art could configure it according to actual needs. As long as the interaction of the limiting structure 302 and the limiting structure 502 facilitates the assembling of the shell 300 to the port 500, it belongs to the gist of the limiting structure.

Advantageously, the inner wall of the shell 300 and the outer wall of the main body 600 are profiled with complementary contours and/or patterns for aiding the assembly between the main body 600 and the shell 300. For example, as illustrated in figure 3A, the shell 300 is internally provided with a limiting structure 304 having shape dimensioned to be complementary to the shape of the limiting structure 602 (or 604) of the main body 600. It shall be important to note that the shapes of the limiting structures 304 and 602 (or 604) disclosed in figures are an example only; those skilled in the art could configure it according to actual needs. As long as the interaction of the limiting structures 304 and 602 (or 604) aids in the assembling between the main body 600 and the shell 300, it belongs to the gist of the limiting structure.

The shaft 400 is at least partially housed inside an accommodation cavity defined by the internal cavity of the main body 600 and the internal cavity of the port 500. The depth of the internal cavity of the port 500 is deeper than the length of the lower portion of the shaft 400, such that the lower portion of the shaft 400 is movable in the internal cavity of the port 500 between the limiting structure 602 (or 604) of the main body 600 and the bottom of the port 500. To be more specific, with the aid of the resilient member 700, disposed between the cap 200 and the main body 600, the lower portion of the shaft 400 is reciprocally movable between the limiting structure 602 (or 604) of the main body 600 and the bottom of the port 500. Advantageously, regardless whether the cap 200 is pressed or not pressed, the bottom of the shaft 400 does not engage with the bottom of the port 500.

The position of the cap 200 with respect to the port 500 controls a pressing action. When a user presses the cap 200, it actuates the lower portion of the shaft 400 to move from a first position where the limiting structure 602 (or 604) of the main body 600 interacts with the limiting structure 402 of the shaft 400 to a second position where the limiting structure 602 (or 604) of the main body 600 does not interact with the limiting structure 402 of the shaft 400. By default, when the cap 200 is unpressed, the cap 200 is at its first position; when the cap 200 is pressed, the cap 200 is at its second position. The position of the cap 200 with respect to the port 500 also defines the configuration of the endoscopic cleaning adapter 100, in which the first position of the cap 200 defines the first configuration of the endoscopic cleaning adapter 100 and the second position of the cap 200 defines the second configuration of the endoscopic cleaning adapter 100.

The shaft 400 features a sophisticated sealing system comprising five precisely machined recesses-designated as a first recess 410, a second recess 420, a third recess 430, a fourth recess 440, and a fifth recess 450-arranged sequentially along the vertical axis of the shaft 400, in which the first recess 410 is at the top. Preferably, the first recess 410 is always in the cavity defined by the main body 600 during the operation of cleaning an endoscope. More preferably, the second recess 420, the third recess 430, the fourth recess 440, and the fifth recess 450 are always in the cavity defined by the port 500 during the operation of cleaning an endoscope. It shall be important to note that the number of recesses provided to the shaft 400 is at least five. Each recess is annularly, preferably continuous, around the tubular structure of the shaft 400 and hosts a plurality of sealing members: a first sealing member 410A, a second sealing member 420A, a third sealing member 430A, a fourth sealing member 440A, and a fifth sealing member 450A, respectively.

Advantageously, the first sealing member 410A is configured as a ring structure having a central bore to sleeve around the first recesses 410. More advantageously, any of the second sealing member 420A, the third sealing member 430A, the fourth sealing member 440A, and the fifth sealing member 450A can be configured to comprise a central bore and an annular protrusion projecting outwardly from the surrounding wall of the central bore. The annular protrusion is sized and dimensioned to act against a side facing opposite to the annular protrusion, such as the inner side of the surrounding wall of the port. It shall be important to note that the annular protrusion is sufficiently long to act against the inner side of the surrounding wall of the port, avoiding contact between the surface of the tubular structure of the shaft and the surface of the inner side of the surrounding wall of the port thus preventing wearing off the surface of the inner side of the surrounding wall of the port. Depending on the application and design, any one of these sealing members 420A, 430A, 440A, and 450A can be configured as any form as long as it can be functioned as a sealing member. As long as the sealing member is sized and dimensioned to act against the inner side of the surrounding wall of the port, avoiding contact between the surface of the tubular structure of the shaft and the surface of the inner side of the surrounding wall of the port thus preventing wearing off the surface of the inner side of the surrounding wall of the port, such alternative of the sealing member also falls within the gist of the invention.

As illustrated in the accompanying figures, the cap 200 is designed with a pair of elongated legs that extend downward from its top surface. These legs are positioned such that they define a cavity or channel between them, which is configured to receive a portion of the upper section of the shaft 400. More specifically, when the endoscopic cleaning adapter 100 is inserted into the port 500, this pair of elongated legs extends from the underside of the cap's top surface in the direction of the port 500.

In certain embodiments, the inner surfaces of these elongated legs are equipped with engagement members such as threaded screws or threads. These threads are designed to engage with corresponding threads (examples of engagement members) provided on the outer peripheral tip or upper end of the shaft 400. This threaded engagement allows the cap 200 to be securely fastened onto the shaft 400, ensuring a stable connection during use. However, it is important to note that the use of threaded screws is merely an example of a possible coupling mechanism. The cap 200 may also be fastened, preferably removably fastened, to the upper portion of the shaft 400 through alternative means, such as a snap-fit connection or a tight-fit engagement, depending on the design requirements or the intended application.

Additionally, the length of the elongated legs serves a functional purpose beyond mechanical engagement. The legs are sufficiently long so that, when the cap 200 is fully installed onto the shaft 400, they apply a downward force onto the first sealing member 410A. This force causes the sealing member to be biased, compressed, or slightly deformed. Such compression enhances the sealing capability of the adapter assembly, ensuring a more secure and reliable connection between the components.

Moreover, the configuration of the elongated legs of the cap 200 and the sealing member 410A provides a significant manufacturing advantage, particularly in terms of enabling automation during the assembly process. Because the design incorporates a simplified mechanical interface-such as threaded engagement, snap-fit, or tight-fit mechanisms-it minimizes the need for complex alignment procedures or the use of specialized tools during installation. This simplification lends itself well to integration with automated assembly lines, where robotic systems or automated machinery can consistently and accurately position and attach the cap 200 onto the shaft 400 with minimal human intervention. By reducing reliance on manual labor, the assembly process becomes not only more efficient but also more scalable and cost-effective.

This is especially beneficial in high-volume production environments, where consistency, speed, and precision are critical. Additionally, automation helps to minimize human error, ensuring a uniform fit and seal quality across all units produced. As a result, the simplified structural design of the cap and its elongate legs directly contribute to improved manufacturing throughput, enhanced product reliability, and lower overall production costs. Furthermore, this approach aligns with modern trends in advanced manufacturing, where the incorporation of automated processes is considered a hallmark of state-of-the-art production systems. By facilitating automation, the design supports streamlined workflows and fosters operational efficiency, which are essential for meeting the demands of contemporary medical device manufacturing standards.

Integral to the shaft's function is an internal channel inside the tubular structure of the shaft 400, which serves as a multifunctional fluid conduit. It functions as a controlled passage for fluids, likely facilitating the directed flow of cleaning fluids during cleaning. The essence of the internal channel is to provide a tunnel with at least a first opening, a second opening, and a hollow cavity connecting with said first and second openings allowing fluidic flow. To be more specific, allowing either water flow or air flow during the endoscopy cleaning procedures. The first opening can be embodied as a first opening 460A or a first opening 460B in figures, while the second opening can be embodied as a first second opening 470A or a second opening 470B in figures. In some embodiments, the hollow cavity 490 is a vertical channel inside the shaft fluidically connecting with the first opening and the second opening. It shall be understood that the first opening provides a first fluidic path for fluid, the second opening provides a second fluidic path for fluid, and the hollow cavity provides a third fluidic path for fluid. When fluid flows from the first opening, it can reach the second opening via the hollow cavity, or vice versa. In some embodiments, the shaft can be configured to provide first opening 460A and/or first opening 460B preferably opposite to the first opening 460A, and also can be configured to provide second opening 470A and/or second opening 470B preferably opposite to the second opening 470A. In some embodiments, when the shaft is provided with both first openings 460A and 460B, it shall be understood that these openings are in fluidic communication thus defining a fluidic path therebetween. In some embodiments, when the shaft is provided with both second openings 470A and 470B, it shall be understood that these openings are in fluidic communication thus defining a fluidic path therebetween.

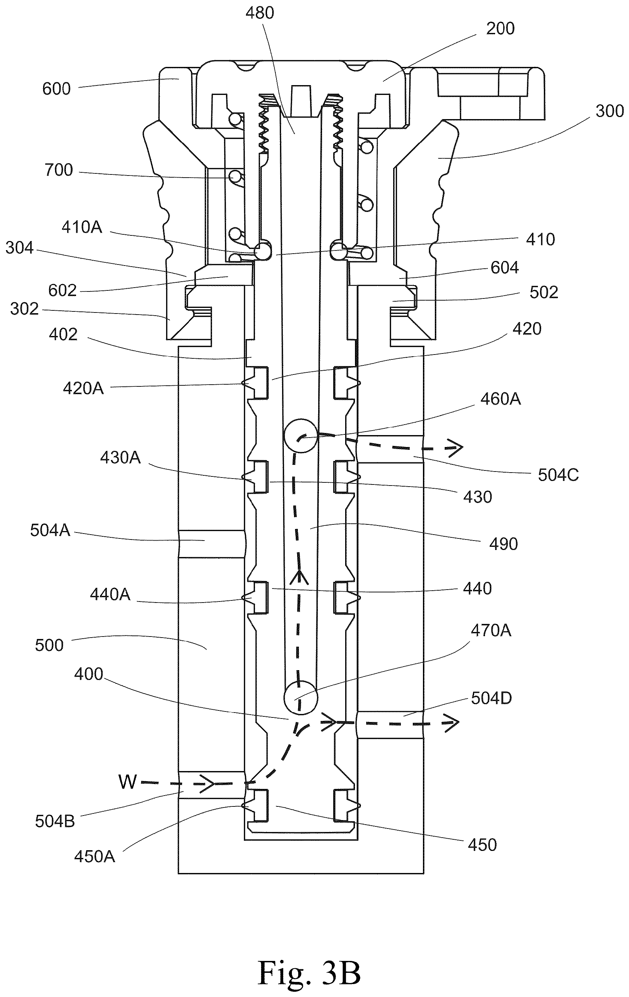

As indicated in the figures, the hollow cavity 490, the first openings 460A and 460B, and the second openings 470A and 470B are in fluidic communication. However, it should be understood that the present invention can be practiced by configuring at least one of the first openings (460A or 460B), at least one of the second openings (470A or 470B), and the hollow cavity 490 in fluidic communication. In other words, the essence of the present invention can be realized by configuring at least one of the first openings 460A or 460B, at least one of the second openings 470A or 470B, and the hollow cavity 490 to be in fluidic communication. Taking figures 3A and 3B as illustration, the internal channel traverses the zones associated with a point - between the second recess 420 and the third recess 430 - and a point - between the fourth recess 440 and the fifth recess 450. Advantageously, the first opening 460A is positioned higher than the third recess 430 and the second opening 470A is positioned higher than the fifth recess 450. It shall be understood that the first opening 460A and the second opening 470A are examples only, those skilled in the art could use the first opening 460B and the second opening 470B. It shall be important to note that although the internal channel is I-shaped in figures, it is for illustration only. As long as an internal channel that has at least two openings, in which one of the two openings is provided on the length side of the tubular structure of the shaft at a position higher than the third recess 430 while another one of the two openings is provided on the length side of the tubular structure of the shaft at a position higher than the fifth recess 450 but below the fourth recess 440, and a hollow cavity fluidically connecting with the at least two openings, it also falls into the gist of the internal channel. In some embodiments, the shape of the internal channel can be Y-shaped, inverted A-shaped, or any shape. In some embodiments, the internal channel has three, four, five or even more openings according to actual needs. In some embodiments, the shaft 400 includes a third opening 480 at its tip, extending from the hollow cavity 490. This third opening 480 can be configured to engage with the cap 200. The third opening 480, the hollow cavity 490, the first opening(s) 460A and/or 460B, and the second opening(s) 470A and 470B can collectively be configured to be in fluidic communication.

The surrounding wall of the port 500 is profiled to provide a first conduit 504A for receiving air flow from an air source for the endoscope, a second conduit 504B for receiving water flow from a water source for the endoscope, a third conduit 504C, and a fourth conduit 504D. Both third conduit 504C and fourth conduit 504D are outlet ports for air and/or water. It shall be important to understand that the use of air and water is for explanation the operation of the endoscopic cleaning adapter and shall not be construed as limitation to the use of the endoscopic cleaning adapter. In some embodiments, the first conduit 504A can be configured for receiving water flow from a water source for the endoscope, a second conduit 504B can be configured for receiving air flow from an air source for the endoscope. It shall also be important to understand that "conduit" in context means a channel or tunnel or through-opening allowing fluid, i.e., liquid and/or air, passing through. "Fluid" in this specification may refer to liquid and/or air. For example, the first conduit 504A is for allowing fluid to pass through the surrounding wall of the port 500. It is important to note that the conduit shall not be constrained as any shape.

When the endoscopic cleaning adapter 100 is mounted to the port 500, the operation of the endoscopic cleaning adapter 100, i.e., pressing or not pressing the cap 200 of the endoscopic cleaning adapter 100, operably allows the first conduit 504A and the second conduit 504B to allow air/water to pass through the endoscopic cleaning adapter 100 to the third conduit 504C and/or the fourth conduit 504D. Subject to cleaning procedures, airflow from the first conduit 504A to the third conduit 504C and/or fourth conduit 504D, optionally through the internal channel, is permitted; and/or water flow from the second conduit 504B to the third conduit 504C and/or fourth conduit 504D, optionally through the internal channel, is permitted. In some cleaning procedures, the cleaning process is specifically performed by passing airflow from the first conduit 504A to the third conduit 504C, and/or to fourth conduit 504D optionally through the internal channel; and/or passing water flow from the second conduit 504B to the third conduit 504C optionally through the internal channel and/or to fourth conduit 504D.

When the endoscopic cleaning adapter 100 is mounted to the port 500, the outer side of the surrounding wall of the shaft 400 and the inner side of the surrounding wall of the port 500 leaves a narrow channel running their entire lengths, which functions as a dedicated fluid passage. In this specification, "narrow channel between the outer side of the surrounding wall of the shaft 400 and the inner side of the surrounding wall of the port 500" and "dedicated fluid passage" are interchangeable. The sealing members 410A, 420A, 430A, 440A, and 450A, made of resilient materials and mounted to their respective recesses of the tubular structure of the shaft 400, acts against the inner side of the surrounding wall of the port 500 thus blocking and/or impeding the narrow channel, i.e., the dedicated fluid passage. It shall be important to understand that "the sealing member acts the inner side of the surrounding wall of the port" in context can also be understood as "the inner side of the surrounding wall of the port biases or presses or compresses the sealing member". When the sealing member is biased, it provides a sealing effect.

Figure 3A depicts the endoscopic cleaning adapter 100 is mounted to the port 500, in which the cap 200 is not pressed, the second sealing member 420A and the third sealing member 430A act against the inner side of the surrounding wall of the port 500, and part of the fourth sealing member 440A does not act against the inner side of the surrounding wall of the port 500. Therefore, during the cleaning step of the endoscope, when the cap 200 is not pressed, air (denoted with letter A in figure 3A) flushes into the first conduit 504A then leaves therefrom, then enters the narrow channel between the exit of the first conduit 504A and the fourth sealing member 440A, then enters the narrow channel between the inner side of the surrounding wall of the port 500 and the outer side of the surrounding wall of the shaft 400. At there, the flushed-in air bifurcates into two paths: the first path - the flushed air flows through the dedicated fluid passage between the outer side of the surrounding wall of the shaft 400 and the inner side of the surrounding wall of the port 500, then leaves the port 500 via the third conduit 504C; and the second path - the flushed air flows through the dedicated fluid passage between the outer side of the surrounding wall of the shaft 400 and the inner side of the surrounding wall of the port 500, then leaves the port 500 via the fourth conduit 504D. Technically, air flows through the internal channel having the first opening 460A and the second opening 470A, although it provides no substantial function and effect in the step of air flushing.

Since the second sealing member 420A and third sealing member 430A act against the inner side of the surrounding wall of the main body 600, it can prevent air from leaving the cavity defined by the lower portion of the shaft 400 and the internal cavity of the port 500 and air can only be directed to flow through the endoscopic cleaning adapter 100 with path as described above.

In figure 3A, during the cleaning step of the endoscope, water flushes into the second conduit 504B. Since the fifth sealing member 450A act against the inner side of the surrounding wall of the port 500, blocking and/or impeding the narrow channel between the outer side of the surrounding wall of the shaft 400 and the inner side of the surrounding wall of the port 500, therefore water fails to pass through the endoscopic cleaning adapter 100 and stops at the second conduit 504B.

Figure 3B depicts that the endoscopic cleaning adapter 100 is mounted to the port 500, in which the cap 200 is pressed and the second sealing member 420A, third sealing member 430A, and fourth sealing members 440A act against the inner side of the surrounding wall of the port 500, respectively. Therefore, during the cleaning step of the endoscope when the cap 200 is pressed, air flushes into the first conduit 504A. Since the third sealing member 430A and the fourth sealing member 440A act against the inner side of the surrounding wall of the port 500, blocking and/or impeding the narrow channel between the outer side of the surrounding wall of the shaft 400 and the inner side of the surrounding wall of the port 500, therefore air fails to pass through the endoscopic cleaning adapter 100 and stops at the first conduit 504A.

In figure 3B, during the cleaning step of the endoscope, water (denoted with letter W in figure 3B) flushes into the second conduit 504B. Since the fifth sealing member 450A is offset from the second conduit 504B, water flows through the narrow channel to fourth conduit 504D through the dedicated fluid passage between the outer side of the surrounding wall of the shaft 400 and the inner side of the surrounding wall of the port 500. At there, the water bifurcates into two paths: the first path - the water flows from the second opening 470A into the hollow cavity 490, then leaves the hollow cavity 490 through the first opening 460A, and then leaves the port 500 via the third conduit 504C; and the second path - the water flows through the dedicated fluid passage between the outer side of the surrounding wall of the shaft 400 and the inner side of the surrounding wall of the port 500, then leaves the port 500 via the fourth conduit 504D. Although this paragraph describes the water flow, with the first opening 460A and the second opening 470A, it shall be understood that the essence of the invention is from the first opening, i.e., the first opening 460A and/or 460B, to the second opening, i.e., the second opening 470A and/or 470B, through the hollow cavity 490.

Due to the novel structural design of the endoscopic cleaning adapter described in the present invention, the shaft can be manufactured as a single, integrated piece with a simplified configuration. This streamlined design enables the use of plastic as the primary material for fabrication, which offers several advantages over conventional designs. In contrast, the shafts used in prior art endoscopic cleaning adapters typically consist of multiple assembled components and are often fabricated from metal. Such multi-piece metal constructions not only increase manufacturing complexity and associated production costs, but also require additional steps for assembly and post-use cleaning. By utilizing plastic materials, the shaft of the endoscopic cleaning adapter of the present invention can be designed for single-use, thereby significantly improving hygiene and reducing the risk of cross-contamination. In some embodiments, the shaft, the cap, the main body all can be fabricated in plastic. Furthermore, the disposable nature of the device eliminates the need for time-consuming cleaning and sterilization procedures associated with reusable metal shafts. This advancement not only simplifies the manufacturing process but also enhances operational efficiency and safety in clinical settings.

The above-described shall not be interpreted to be restricted by the examples or figures only. It is to be expressly understood, however, that such modifications and adaptations are within the scope of invention in this aspect. For instance, features illustrated or described as part of one embodiment can be used on another embodiment to yield a still further embodiment. Thus, it is intended that the present disclosure cover such modifications and variations and their equivalents.

## Claims

1. An endoscopic cleaning adapter, adaptable to position in a port of an endoscope for assisting a cleaning procedure for said endoscope, comprising:
a main body, comprising an internal cavity and a limiting structure, and being positioned on said port when said valve being positioned in said port;
a shaft, partially received in said internal cavity of said main body and profiled into a tubular structure having
- at least five recesses on the length side thereof, wherein each of said at least five recesses being fitted with a sealing member;
- at least two openings on the length side thereof and a hollow cavity fluidically connecting with said at least two openings defining an internal channel inside said tubular structure allowing fluid passing therethrough; and
- a limiting structure;
a shell, adjacent said main body, for fastening said adapter to said port;
**characterized in that**, said adapter further comprising:
a cap removably engaging with said shaft and having an elongate member biasing a sealing member of a first recess of said at least five recesses of said shaft;
a resilient member between said cap and said main body, whereby
said shaft being adapted to be movable reciprocally between a first position where said limiting structure of said shaft engaging said limiting structure of said main body and a second position where said limiting structure of said shaft disengaging said limiting structure of said main body.

2. The adapter according to claim 1, wherein an engagement member provided to said shaft engaging with an engagement member provided to said elongate member of said cap.

3. The adapter according to claim 1, wherein said shaft being manufactured as a single piece.

4. The adapter according to claim 1, wherein said shaft being manufactured in plastic.

5. The adapter according to claim 1, wherein said cap, said main body, and said shell being made of plastic.

6. The adapter according to claim 1, when said shaft being positioned in said port: -
- when said shaft at said first position, water flow in said cleaning procedure being impeded by said adapter; and
- when said shaft at said second position, air flow in said cleaning procedure being impeded by said adapter.

7. The adapter according to claim 6, said adapter being configured to enable: -
- when said shaft being at said first position, air flow, flowing into said port via a first conduit of said port, leaving said port via a third conduit and/or a fourth conduit of said port through fluid passage between the outer side of the surrounding wall of said shaft and the inner side of the surrounding wall of said port; and
- when said shaft being at said second position, water flow, flowing into said port via a second conduit of said port, leaving said port via said third conduit through said internal channel of said shaft and said fourth conduit through fluid passage between the outer side of the surrounding wall of said shaft and the inner side of the surrounding wall of said port.

8. The adapter according to claim 7, wherein when said adapter being positioned in said port, and
when said shaft being at said first position,
a first portion of air flow flowing into said port, via said first conduit, and leaving said port via said third conduit through fluid passage between the outer side of the surrounding wall of said shaft and the inner side of the surrounding wall of said port; and/or
a second portion of air flow flowing into said port, via said first conduit, and leaving said port via said fourth conduit through fluid passage between the outer side of the surrounding wall of said shaft and the inner side of the surrounding wall of said port; and/or
when said shaft being at said second position,
a first portion of water flow flowing into said port, via said second conduit, and leaving said port via said third conduit through said internal channel of said shaft; and/or
a second portion of water flow, flowing into said port, via said second conduit, and leaving said port via said fourth conduit through fluid passage between the outer side of the surrounding wall of said shaft and the inner side of the surrounding wall of said port.

9. The adapter according to claim 1, wherein said shell comprising a limiting structure collaboratively working with a limiting structure of said port for fastening said main body to said port.

10. The adapter according to claim 1, wherein when said shaft being positioned in said port, said limiting structure of said main body and said limiting structure of shaft holding said shaft at least partially received in an accommodation cavity defined by said internal cavity of said main body and an internal cavity of said port of said endoscope.

11. The adapter according to claim 1, wherein said at least five recesses comprising said first recess, a second recess, a third recess, a fourth recess, and a fifth recess sequentially arranged from the top to said bottom of said shaft on the length side thereof.

12. The adapter according to claim 11, wherein one of said at least two openings of said shaft being provided between said second recess and said third recess, while another one of said at least two openings of said shaft being provided between said fourth recess and said fifth recess.

13. The adapter according to claim 1, wherein
said tubular structure being provided with at least a first opening, a second opening, a third opening, and a fourth opening, wherein
said first opening and said second opening defining a first fluidic path, said third opening and said fourth opening defining a second fluidic path, said hollow cavity fluidically connecting with said first fluid path and said second fluidic path.

14. The adapter according to claim 11, wherein
said tubular structure being provided with at least a first opening, a second opening, a third opening, and a fourth opening, wherein
- said first opening and said second opening defining a first fluidic path, said third opening and said fourth opening defining a second fluidic path, said hollow cavity fluidically connecting with said first fluid path and said second fluidic path.

15. The adapter according to claim 14, wherein said first fluidic path being positioned between said second recess and said third recess, while said second fluidic path being positioned between said fourth recess and said fifth recess.
